**Europäisches Patentamt**

**European Patent Office**

**Office européen des brevets**

(19)

(11) Publication number: **0 102 661**
**A1**

(12) # EUROPEAN PATENT APPLICATION

(21) Application number: **83201153.0**

(22) Date of filing: **03.08.83**

(51) Int. Cl.³: **C 12 N 15/00**
**C 12 Q 1/68, G 01 N 31/22**

(30) Priority: **04.08.82 NL 8203102**

(43) Date of publication of application:
**14.03.84 Bulletin 84/11**

(84) Designated Contracting States:
**AT BE CH DE FR GB IT LI LU NL SE**

(71) Applicant: **Katholieke Universiteit, Faculteit der Wiskunde en Natuurwetenschappen Toernooiveld 1 NL-6525 ED Nijmegen(NL)**

(72) Inventor: **Saris, Christiaan J. M. The Salk Institute Tumor Virology Lab. P.O. Box 85800 San Diego, CA. 92138(CA)**

(74) Representative: **Urbanus, Henricus Maria, Ir. et al, c/o Vereenigde Octrooibureaux Nieuwe Parklaan 107 NL-2587 BP 's-Gravenhage(NL)**

(54) A paper for RNA blotting; a process for making same; applications of the paper.

(57) RNA blotting paper; a process for making same, a process for blotting RNA, a process for releasing RNA from a blot, a process for converting mRNA into protein by translation, a process for converting RNA into DNA by reverse transcription, a process for analyzing a mixture of RNAs by applying electrophoresis and a process for detecting RNA in blots by hybridization, wherein the blotting paper can bind RNA through ion exchange groups in a reversible fashion by electrostatic interaction.

EP 0 102 661 A1

Croydon Printing Company Ltd

Title:

A paper for RNA blotting; a process for making same;
applications of the paper.

The invention relates to a paper for RNA blotting, as well
as to a process for making same and to various applications of the
paper.

For some years molecular biology has been  enriched by a
new technique enabling the transfer of nucleic acids contained in a
gel to a solid carrier.
This technique designated by "blotting" has been developed by E.M.
Southern, J.Mol.Biol. 38 (1975), pp. 503-517, who employed a cellulose
nitrate filter as a carrier and could transfer thereto DNA from a gel.
J.C. Alwine et al., Proc.Natl.Acad.Sci. USA 74 (1977), pp. 5350-5354
and Methods in Enzymology 68, (1979), pp. 220-242, realized the transfer of RNA from gels by using as a carrier a chemically reactive
paper to which the RNA is bound in a covalent fashion. The reactive
paper is made by diazotization of aminobenzyl oxymethyl paper, which
itself is made from Whatman 540 paper. For a better understanding of
the blotting technique and its meaning, reference is made to R.W. Old
and S.B. Primrose, Principles of Gene Manipulation, an introduction to
Genetic Engineering, second edition, Studies in Microbiology, Vol. 2,
Blackwell Scientific Publications, 1981, chapter I, pp. 5-9.

In the prior art blotting procedures the characterization of
the nucleic acids transferred to a solid carrier is limited to detection by hybridization with previously isolated radioactive nucleic
acids (radioactive probes).

For hybridization it is required that the nucleic acids be firmly linked with the solid carrier by covalent bonds. Owing to these strong covalent bonds, which have been formed by reactions between the nucleic acids and the solid carrier, it is not possible to release the nucleic acids from the blot without destruction of the nucleic acids themselves.

It is an object of the invention to present a paper for RNA blotting which is devoid of the above indicated problem of limited usability.

According to the invention this object is achieved by means of a paper for RNA blotting, which is characterized in that the paper contains ion exchange groups of such a nature and in such amounts per unit of area that RNA can be bound in a reversible fashion by electrostatic interaction.

By the term "reversible" is meant the possibility of releasing the bound RNA again from the paper without destruction of the RNA.

Although, in principle, there is a wide selection from available types of paper and ion exchange groups, it has further been found that a very suitable paper for RNA blotting is characterized in that the ion exchange groups are introduced by treating paper or paper starting material with NaOH, triethanolamine and epichlorohydrin.

The paper or paper starting material (cellulose powder) which is subjected to this treatment with NaOH, triethanolamine and epichlorohydrin, is preferably Whatman 1MM paper or the starting material therefor.

It is further preferred that in the treatment of the paper or paper starting material a mixture of triethanolamine and epichlorohydrin is used having a volume ratio triethanolamine/epichlorohydrin of 1 : 2.5 to 1 : 3.5.

The best results are obtained if the volume ratio triethanolamine/epichlorohydrin in the mixture is 1 : 2.8 to 1 : 3.3.

In the preceding paragraphs reference has been made to the treatment of paper or paper starting material. By this are meant the possibilities of subjecting, on the one hand, ready paper, i.e. which is already in the form of paper sheets, to the above treatment, and, on the other hand, paper starting material, i.e. material which has not yet been brought into the form of paper sheets such as cellulose powder, to the relevant treatment and processing the resulting product to paper sheets, according to a known per se method.

A treatment of cellulose powder with NaOH, triethanolamine and epichlorohydrin, however, is known per se from E.A. Peterson and H.A. Sober, J. Amer.Chem.Soc. 78 (1956), pp. 751-755. This publication deals with ion exchange groups-containing adsorbents on the basis of cellulose which are useful for the chromatography of proteins. Among the tested celluloses provided with ion exchange groups (there is no question of making paper) is also the so-called ECTEOLA cellulose, which is said to be suitable not so much in the protein adsorption as in the chromatography of nucleic acids. The name refers to the employed reaction components epichlorohydrin and triethanolamine because the structure of the ion exchange groups introduced into the cellulose is unknown.

ECTEOLA paper made by using the method described in this publication proves to be unsuitable as a paper for RNA blotting because the binding of the RNA, larger than 10S, is much too strong so that it is impossible to release the RNA from the blot without destroying the RNA. The same drawback is also connected with commercially available ion exchange groups-containing paper types such as DEAE cellulose and Servacel WB-2. By modifying the procedures according to which these paper types are made, however, the binding characteristics of the paper relative to RNA can be altered in the desired direction.

The invention also relates to a process for making a paper for RNA blotting, which is characterized in that a paper or paper starting material is provided with ion exchange groups of such a nature and in such amounts per unit of area that RNA can be reversibly bound by electrostatic interaction.

Although a wide range of types of paper and ion exchange groups is available, it is especially preferred that the ion exchange groups be introduced by treating paper or paper starting material with NaOH, triethanolamine and epichlorohydrin, as well as that the treatment be carried out at Whatman 1MM paper or the starting material therefor.

A very suitable paper for RNA blotting is obtained if in the treatment of the paper or paper starting material a mixture of triethanolamine and apichlorohydrin is used having a volume ratio triethanolamine/epichlorohydrin of 1 : 2.5 to 1 : 3.5.

The best results are obtained if the volume ratio triethanolamine/epichlorohydrin in the mixture is 1 : 2.8 to 1 : 3.3.

A more concrete preferred embodiment of the process for making paper for RNA blotting comprises the steps of immersing Whatman 1MM paper sheets in an aqueous NaOH solution, removing excess NaOH through a yield treatment, then immersing the sheets in a mixture of triethanolamine and epichlorohydrin, in which the volume ratio triethanolamine/epichlorohydrin is 1 : 2.5 to 1 : 3.5, preferably 1 : 2.8 to 1 : 3.3, removing excess mixture through a yield treatment, then immersing the sheets in an aqueous NaOH solution and heating the sheets therein, and subsequently stopping the reaction, washing and finally drying the sheets.

In this process it is preferred that the aqueous NaOH solution employed is an about 10 M NaOH solution, and that the first treatment with NaOH is carried out for about 30 minutes at about 0°C, that the second treatment with NaOH is carried out for about 2 hours at about 85-90°C, that the treatment with a mixture of triethanolamine and epichlorohydrin is carried for about 15 minutes at room temperature, that the reaction is stopped by placing the sheets in ice-cold 3M KAc-HAc buffer pH 7.4, that the sheets are washed a few times alternately with 1M HAc and 1M KOH, then with water until neutral and finally with absolute ethanol, and that the sheets are air-dried.

The invention also relates to various applications of the new paper according to the invention, as there is first of all its use as blotting paper for blotting RNA. The blotting technique is known per se from, inter alia, some earlier-mentioned publications.

When RNA is blotted by using the paper according to the invention as blotting paper (i.e. as a solid carrier for the RNA), the transferring agent preferably used is a buffer liquid having a moderate ionic strength (relatively low salt concentration) and an approximately neutral pH. Very suitable as transferring liquid is the buffer 100 mM KAc-HAc pH 7.4. In the presence of such a buffer having a relatively low salt concentration the RNA is firmly and quantitatively bound to the blotting paper by electrostatic interaction.

A significant advantage of the blotting paper according to the invention is that the bound RNA may be released from the paper again by modifying the conditions so that it can be used for further procedures.

The invention therefore also relates to a process for releasing RNA from paper to which it has been bound by blotting, which process is characterized in that it is released by elution from an RNA blot obtained by using the new paper according to the invention.

The eluting agent preferably employed is a liquid having high ionic strength and basic pH. Use is preferably made of a volatile liquid so that the RNA can be simply recovered therefrom by lyophili-sation in a form suitable for further experiments. If a non-volatile liquid is used, the RNA can be precipitated therefrom with ethanol indeed, but it has the disadvantage that relatively large amounts of carrier material must be added which could have a certain effect on further experiments.

30% Triethylamine carbonate (TEC) pH 9.0 has proved to be a very suitable eluting agent.

The RNA recovered from the RNA blots can be used for various further procedures. In this connection special thought is given to in vitro (cell-free) translation of messenger RNA in protein by means of a translation mixture which contains all the required components such as ribosomes and many other RNA and protein factors, as well as to reverse transcription of the RNA into DNA by means of the enzyme reverse transcriptase, after which the thus obtained cDNA (copy DNA) may serve as the starting-point for genetic manipulations. Such applications rendered possible by the invention are also comprised.

Surprisingly, it has appeared that messenger RNA blots according to the invention are also suitable for translation in protein, without the RNA being first released from the paper for that purpose. This is immediately connected with the fact that the RNA is bound to the paper only by relatively weak electrostatic interactions, allowing the translation mechanism, in which ribosomes and messenger RNA chain must be able to move along each other, to proceed freely. In such an in vitro translation of the paper-bound RNA (in situ translation) also the translation products (proteins) are surprisingly bound to the paper in situ.                This property opens the possibility of blotting proteins contained in gels with the paper according to the invention and then examinating, e.g., their enzymatic activity. This property also creates the possibility of a completely new two-dimensional analysis of a mixture of messenger RNAs, which will be elucidated hereinafter.

The invention therefore comprises a process for converting messenger RNA into protein by translation, which is characterized in that the translation is carried out by incubating in a translation mixture a messenger RNA blot obtained by using the new paper according to the invention.

It has been found that many incorrect translation products, substantially premature terminations, are obtained when the paper-bound RNA is immersed and incubated in the translation mixture directly after transfer from the gel. After many unsuccessful attempts to improve the translation accuracy by altering the concentrations of individual constituents of the translation mixture, it has been found that the translation proceeds with great fidelity when the RNA blot is first saturated with translation mixture by immersing the blot in complete translation mixture without the radioactive amino acids. It seems that binding sites on the paper are first saturated with certain factors to prevent depletion of the translation mixture.

The thus obtained paper-bound proteins may then be released from the paper in a simple manner, e.g., by washing with an electrophoresis sample buffer (e.g., 10M urea, 62.5 mM Tris-HCl pH 6.8, 3% SDS, 5% 2-mercaptoethanol), after which they could be analyzed, e.g., by immunoprecipitation.

The invention further comprises a process for analyzing a mixture of messenger RNAs by applying electrophoresis, which is characterized by carrying out an analysis in two dimensions by electrophoresis of the mixture of messenger RNAs in one dimension, blotting of the separated messenger RNAs by using the new paper according to the invention, in situ translation of the messenger RNAs in protein, and electrophoresis of the resulting proteins in the second dimension.

The separation of the messenger RNAs is preferably effected by agarose gel electrophoresis, while the proteins are preferably separated by sodium dodecylsulfate polyacrylamide gel electrophoresis (SDS-PAGE).

Just as the blots which have been obtained by using known blotting paper types, so can the RNA blots obtained by using the new paper according to the invention be subjected to a hybridisation. However, because the electrostatic interactions by which the RNA is bound to the paper according to the invention are not resistant to the conditions prevailing in hybridisation, the binding of the RNA to the paper must first be strengthened by introducing covalent bonds. It has been found that this can be simply effected by introducing phosphodiester bonds between terminal phosphate groups of the RNA and hydroxyl groups of the paper by means of carbodiimide, in accordance with the procedure described by P.T. Gilham, Biochem. 7 (1968) pp. 2809-2813. The problem of the formation in this reaction of 2'-3' cyclic phosphate can be overcome by generating 5' phosphate groups, for which purpose a limited hydrolysis of the bound RNA to a limited number of fragments is carried out with P1 nuclease.

It is also possible to effect a bond between the RNA and the paper sufficiently strong for hybridisation purposes by heating the blot in vacuo after it has been dried completely, e.g., for 10 minutes at 50°C. The hybridisation efficiency, however, is then clearly lower.

These hybridisation applications of blots obtained by using the new paper according to the invention are likewise comprised here.

Finally, it is speculated that besides in situ translation also reverse transcription and further reactions involved in cloning cDNA may be carried out in situ, i.e. at the RNA blot itself.

The invention will be further elucidated by means of the examples given hereinbelow.

Example I

Preparation of modified ECTEOLA paper

The reaction protocol described here is based on the procedure of Peterson and Sober. Several adaptations, however, were needed to make the paper suitable for the specific purpose of blotting large RNA molecules. Since blotting requires sheets of the solid carrier instead of a suspension (for which the published procedure is described), a number of problems connected with this aspect had to be solved. Firstly, a number of different types of paper was tested because the source of the cellulose (wood or cotton) strongly influences the reactivity. Of the paper types tested, Whatman 1MM gave the best results. Furthermore, when using paper sheets, the reaction mixture does not remain homogeneous because it cannot be stirred vigorously as is the case with cellulose powder. This problem was overcome by altering the sequence in which the reactants were added. Finally, a large number of different ratios of the reactants were tested to yield a paper with the desired binding characteristics. Binding criteria were: immobilisation of (ribosomal) RNA in 100 mM KAc-HAc pH 7.4 and release in 30% TEC pH 9.0. The resulting protocol is as follows: a sheet of Whatman 1MM paper was immersed in 10 M NaOH in a Pyrex oven dish for 30 minutes at 0°C. Excess NaOH was blotted by firmly pressing the sheet between two layers of Whatman 3MM paper. Pressure was applied with a rolling device, commonly used in the drying of photographic prints. The sheet was then immersed for 15 minutes at room temperature (fume hood) in a reaction mixture which typically consisted of 8.1 g (about 7.2 ml) triethanolamine (Merck, Darmstadt, Federal Republic of Germany) and 22.5 ml epichlorohydrin (Merck). Excess reaction

mixture was blotted as described before for NaOH, and the sheet was placed back in the Pyrex dish with 10 M NaOH. The dish was transferred to a waterbath and kept at 85-90°C for 2 hours. Care was taken that the sheet remained fully submerged in NaOH during the reaction by placing glass weights along the edges. During the reaction the sheet should not turn yellowish which occasionally did happen when excess reaction mixture had not been blotted off sufficiently. Yellowish paper appeared    to bind RNA too strongly. The reaction was stopped by placing the sheet in ice-cold 3M KAc-HAc pH 7.4, followed by three successive washings in 1M HAc and 1M KOH. Finally, the paper was washed until neutral and air dried after washing in absolute ethanol.

Example II

Blotting

Moloney murine leukemia virus (M-MuLV) 70S RNA was isolated from virions (harvested at 2 hours or 12 hours intervals) by Proteinase K digestion and sucrose gradient centrifugation. Cytoplasmic RNA from virus-infected tissue culture cells was isolated by magnesium-precipitation according to the procedure of R.D. Palmiter, Biochem. 13, (1974), pp. 3606-3615, followed by oligo dT-cellulose (Collaborative Research, T-3 grade) chromatography. RNA was separated on 2 mm thick vertical 1% acid urea agarose gels according to the procedure described by J.M. Rosen et al., Biochem. 14, (1975), pp. 69-78. After electrophoresis the RNA was blotted onto the paper made according to Example I by applying the procedure described by Southern using 100 mM KAc-HAc pH 7.4 as transfer buffer. Transfer was used for 15 hours at 4°C, although transfer is probably complete in a much shorter time. The resulting

0102661

blots were washed in transfer buffer and stored until further use under 1 volume of transfer buffer and 2 volumes of ethanol at -20°C.

Example III

Blot hybridisation

For covalent binding of RNA to the paper the blot was first treated with P1 nuclease to generate 5'-phosphates. The blots were washed in P1 buffer (10 mM NaAc-HAc pH 5.3, 0.4 mM $ZnSO_4$, 4 mg/ml bovine serum albumin) and incubated for 1 hour at 37°C in 15 $\mu$l/cm$^2$ of P1 buffer containing $10^{-3}$ units/ml P1 nuclease (Boehringer, Mannheim) and 33 $\mu$g/ml rabbit tRNA. Subsequently, the blot was washed in 20 mM sodium 2-(N-morpholino) ethanesulfonate (MES) pH 6.0, 5 mM EDTA, followed by extensive washing in water and complete drying in vacuo at room temperature. Then the blot was gently agitated for 24 hours at room temperature in excess carbodiimide reaction mixture, typically prepared by dissolving 150 mg N-cyclohexyl-N'-ß-(4-methylmorpholinium) ethyl carbodiimide p-toluenesulfonate (Merck) in 0.3 ml 0.2 M MES pH 6.0 and 2.4 ml water. After the reaction the blot was briefly washed in, successively, dilute ammonia having a pH of 9.0; 5 mM HEPES pH 7.5; and prehybridisation buffer. Hybridisation to $^{32}$P nick-translation labelled probes (see Old and Primrose, pp. 97-98) took place at 41°C for 16 hours, essentially as described by B. Adkins et al., J. Virol. 41, (1982), pp. 767-780.

Hybridisation to M—MuLV specific cDNA (prepared according to H. Van der Putten et al., Cell. 24 (1981), pp. 729-739) took place at 68°C for 4.5 hours in 10 mM TES pH 7.4, 1 mM EDTA, 1 M NaCl, 0.2% SDS, 2 µg/ml polyvinylchloride, 1 mg/ml denatured calf thymus DNA, 10 µg/ml poly A, 5x Denhardt solution, 20 mM phosphate, followed by successive washing in 50% formamide, 5x SSC, 1% SDS and 2x SSC, 1% SDS at 41°C, and 0.1x SSC, 0.1% SDS at 50°C.

## Example IV

## Blot translation and SDS-PAGE

Strips or sections of blot were washed in 100 mM KAc-HAc pH 7.4, blotted between Whatman 3MM paper and saturated (5 min. at 0°C) with excess complete translation mixture, only lacking radioactive amino acid(s). The blot was again firmly pressed between 2 layers of 3MM paper and incubated in 33 µl/cm² of complete translation mixture for 2 hours at 30°C. Small paper sections were incubated in 0.5 ml Eppendorf vials, entire strips in sealed plastic bags. The in situ translation products were released from the paper in electrophoresis sample buffer (10 M urea, 62.5 mM Tris-HCl pH 6.8, 3% SDS, 5% 2-mercaptoethanol) and analyzed on 10% polyacrylamide slab gels according to the discontinuous system of U.K. Laemmli, Nature 227 (1970), pp. 680-685.

For SDS-PAGE of entire strips, the strips were sealed in low melting agarose (Sea-plaque) in the gel slot after washing in 62.5 mM Tris-HCl pH 6.8, 5% 2-mercaptoethanol. The cell-free translation system used was the mRNA dependent reticulocyte cell-free system, prepared essentially as described by H.R.B. Pelham and R.J. Jackson, Eur. J.Biochem. 67, (1976), pp. 247-256, except that white blood cells were removed by chromatography over SP-Sepharose according to the method of M. Perucho et al., Anal.Biochem. 98 (1979), pp. 464-471.

The reticulocyte lysates were treated with micrococco nuclease in the presence of 2 mM DTT, 10 µg/ml hemin, 50 µg/ml creatine kinase and stored at -70°C. Typical translation mixtures (final volume 200 µl) contained 120 µl of reticulocyte lysate and (added) final concentrations of 100 µg/ml creatine kinase, 10 mM creatine phosphate, 0.3 mM spermidine-HCl, 0.1 mM of each unlabeled amino acid (always including glutamine), 50 µg/ml rabbit tRNA, 1 mM MgAc$_2$, 100 mM KAc, 1 mM ATP, 0.2 mM GTP in addition to one or more radioactive amino acids.

Example V

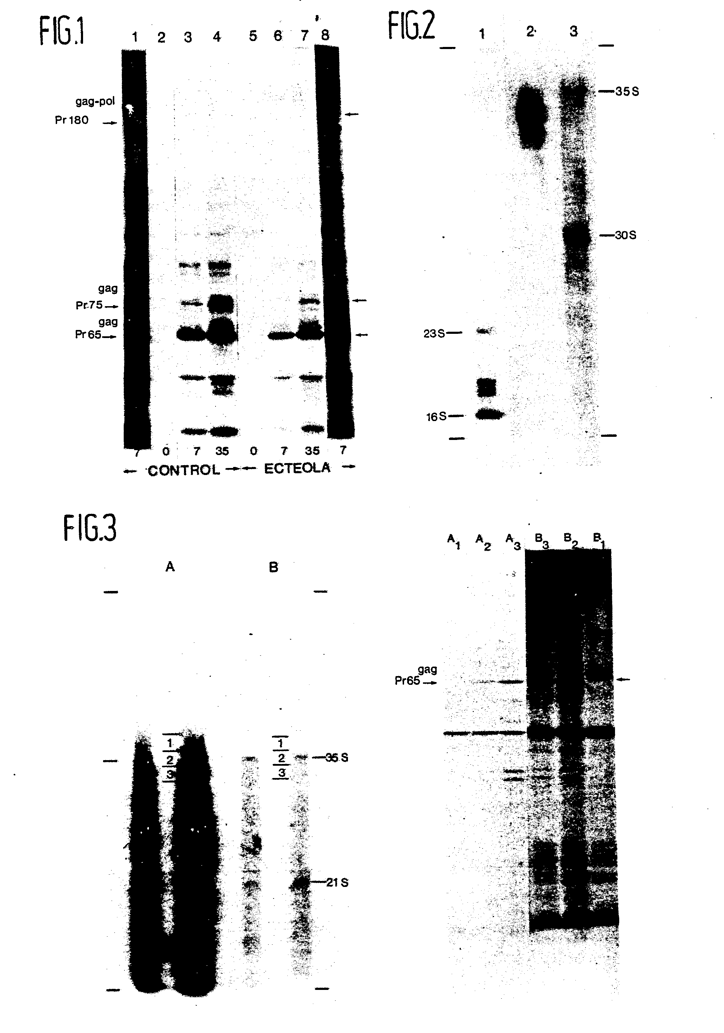

A comparison was made between the translation products of increasing concentrations of Moloney murine leukemia virus (M-MuLV) 70 S virion RNA, which comparison took place between the translation products obtained by translation in solution, on the one hand, and the translation products obtained by translation in situ on paper according to the invention, on the other hand. For this comparison, reference is made to Fig. 1, in which lanes 1-4 show the result of translation in solution and lanes 5-8 show the result of translation in situ on the paper according to the invention.

Fig. 1 shows an SDS-PAGE analysis of cell-free translation products of M-MuLV 70 S virion RNA. RNA dissolved in water in increasing concentrations was either added to a reticulocyte translation mixture or was spotted on 3x3 mm paper sections as made in Example I. The translation was performed in a final volume of 10 ul, of which 3 ul was applied to the gel after mixing with 20 ul of electrophoresis sample buffer. The paper sections according to the invention were washed in 100 mM KAc-HAc pH 7.4 to remove unbound RNA, if any, and prepared for in situ translation as described in Example IV. Translation in 3 ul of complete mixture per 9 mm² of paper according to the invention was stopped by addition of 20 ul sample buffer to elute immobilized translation products. The supernatant (23 ul), leaving behind the paper, was applied to the gel. The final RNA concentrations in ug/ml are indicated at the bottom of each lane. Lanes 1 and 8 are longer exposures of lanes 3 and 6, respectively.

The autoradiogram of the analytical SDS gel shows that the in situ translation yields a set of products which is virtually indistinguishable from the set synthesized in solution, but with a small reduction in the amount of high molecular weight poypeptides. It has been found that the small reduction in high molecular weight proteins is not due to the quality of the translation, but to their retention on the ion exchange paper. This problem can easily be overcome by including the paper piece in the slot of the analytical SDS gel. It has also been found that the in situ translation efficiency can be slightly increased by applying the RNA to the paper according to the invention in a detanuring buffer (the same buffer as used for the RNA gel electrophoresis) instead of in water. The in situ translation of RNA bound to the paper according to the invention is therefore highly accurate and makes elution of the RNA from the paper for this purpose superfluous.

## Example VI

## Blot hybridization

In order to show the detection of specific RNA by hybridization, purified M-MuLV 35S virion RNA, poly A selected cytoplasmic RNA of Moloney sarcoma virus (MSV) transformed M-MuLV infected cells, and $^{32}$P labeledRNA from M13 infected E.coli cells were electrophoresed on acid urea agarose gels according to Rosen et al. and blotted onto paper according to the invention obtained according to Example I. After limited P1 nuclease digestion and carbodiimide coupling the blot was divided in two sections and autoradiographed either directly or after hybridization to $^{32}$P labeled M-MuLV specific cDNA. The resulting autoradiograms are shown in Fig. 2, in which lane 1 shows the direct search and lanes 2 and 3 show the search after hybridization. The RNAs were: $^{32}$P labeled RNA from M13 infected E. coli (lane 1); sucrose gradient purified M-MuLV 35S virion RNA (isolated from virions harvested at 2 hours intervals) (lane 2); and poly A selected cytoplasmic RNA from Clone 124 MSV transformed M-MuLV infected cells (lane 3).

The bands at 35S (M-MuLV) and at 30S (MSV) were detected specifically against a low background hybridization. The sharp marker bands in lane 1 indicate that no spreading of the RNA takes place during the P1 nuclease digestion and the carbodiimide reaction. The sensitivity of detection is comparable to what can be obtained with blotting onto nitrocellulose according to the procedure of P.S. Thomas, Proc.Ntal. Acad. Sci. USA 77, (1980), pp. 5201-5205.

## Example VII

### Correlation of translation and hybridization profiles

To demonstrate the possibility of accurately correlating in situ translation products with specific bands in the hybridization pattern, poly A selected cytoplasmic RNA from tissue culture cells and retroviral virion RNA were analyzed. The RNA was separated on an acid urea agarose gel and transferred to two layers of paper according to the invention. The lower layer consisted of two separate (1x11 cm) strips which were placed on top of and in direct contact with the gel, each covering the lengthwise central section of one (2 cm wide) lane. The strips were notched along one side to allow accurate alignment of paper sections used for in situ translation with the hybridization profile of an upper layer of paper according to the invention (6x11 cm) which covered the two strips and the rest of the gel. This way, RNA migrating within the central 1 cm of each lane was transferred to the lower layer of paper according to the invention (the strips) whereas material migrating along the edges (0.5 cm on either side) was transferred to the upper layer. To prevent degradation of transferred RNA, the layers were stored until further use under 70% ethanol, 33 mM KAc at -20°C, immediately after transfer. After treatment with P1 nuclease and carbodiimide, the upper layer was hybridized with a $^{32}$P nick translation labeled probe and autoradiographed. For the autogram reference is made to Fig. 3 left panel.

The left panel of Fig. 3 shows a lane A and a lane B. M-MuLV 70 S virion RNA isolated from virions harvested at 12 hours intervals (lane A), and poly A selected cytoplasmic RNA from AbLV transformed M-MuLV infected cells (lane B) were electrophoresed on a 1% acid urea agarose gel and transferred to.two layers of paper according to the invention. The upper layer was hybridized to $^{32}$P nick translation labeled cloned M-MuLV proviral DNA (pMLV). Fig. 3 shows the autoradiogram in the left panel.

The right panel shows an SDS-PAGE analysis of in situ translation products. The lower layer strips of paper according to the invention were lengthwise cut in half and the notched half was subdivided in 4x4 mm sections (spanning a single notch each). Sections indicated in the left panel were analyzed by SDS-PAGE after in situ translation.

The RNA in lane B had been isolated from Abelson leukemia virus (AbLV) transformed ANN cells which were productively infected with M-MuLV. Although the AbLV genome contains sequences related to M-MuLV, the probe (cloned proviral M-MuLV DNA; pMLV) does not clearly detect AbLV specific 30 S RNA. This is probably due to the relatively low concentrations of this RNA species and to the nature of the probe (M-MuLV specific instead of AbLV specific, and nick translation labeled cloned DNA instead of cDNA). In general cDNA probes (as used in Fig. 2) gave better results than nick translation labeled probes.

M-MuLV specific RNA was detected as discrete bands at 35S (genomic RNA, gag mRNA and gag-pol mRNA) and at 21S (env mRNA). The faint background hybridization within the lane, starting at 35S, indicates that breakdown of RNA occurred prior to or during electrophoresis.

The native M-MuLV 70S virion RNA (lane A) is a dimer of 35S genomic RNA, complexed with some minor contaminants of both viral and cellular RNAs. As was to be expected for total RNA isolated from virions harvested at 12 hours intervals, the 35S RNA was highly fragmented. The absence of hybridization in the centre of both lanes A and B indicates that RNA from this part of each lane was quantitatively retained by the lower layer strips of paper according to the invention in the transfer procedure.

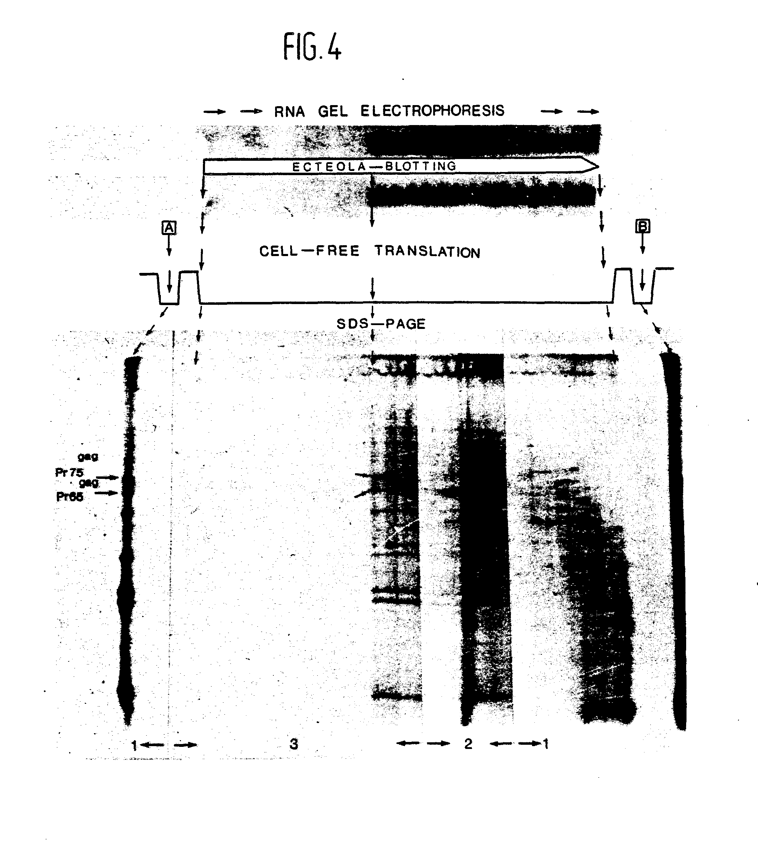

These lower layer strips were cut in half lengthwise and translated in situ as described above for spotted RNA. The smooth edge half was translated entirely and the further analysis of it, which is shown in Fig. 4, will be discussed later. The notched half was subdivided as indicated in Fig. 3 (left panel) and individual sections, each spanning a single notch, were translated. Product analysis by SDS-PAGE of sections surrounding the position of 35S is shown in Fig. 3, right panel. It is clear that the onset of the synthesis of 35S RNA encoded Pr65$^{gag}$ coincided with the position of 35S RNA in the hybridization profile. Thus it may be concluded therefrom that the blotting procedure according to the invention allows translation products to be accurately correlated with specific RNAs.

Example VIII

A two-dimensional version of the blotting procedure is shown in Fig. 4, where translational products of the entire smooth edge half of the lower layer strip of lane B in Fig. 3, left panel, were analyzed by SDS-PAGE. After translation incubation in a sealed plastic bag, the strip was washed and placed horizontally in a wide slot in the gel. To prevent horizontal spreading of protein by direct contact of the strip with electrophoresis sample buffer, the strip was first sealed in the slot with low melting agarose which, after solidifying, was overlayered with sample buffer and electrophoresis buffer. In separate small slots on either side of the wide slot, sections of paper according to the invention with in situ translational products were placed, onto which M-MuLV virion RNA (A) and poly A RNA from AbLV/ M-MuLV infected ANN cells (B) had been spotted.

Fig. 4 shows the SDS-PAGE analysis of in situ translational products of an entire top to bottom strip of the blot of which the hybridization profile is shown in Fig. 3, left panel, lane B, and which is again shown horizontally at the top of this figure. The smooth edge strip was cut from the lower layer notched strip and prepared for in situ translation and subsequent SDS-PAGE analysis as described above. The RNA preparations which were analyzed by gel electrophoresis in the left panel of Fig. 3, lanes A and B, were also spotted on 4x4 mm sections of paper according to the invention and their in situ translational products were run alongside those of the horizontal strip in this figure. The autoradiogram was composed of different exposures of gel sections which are indicated at the bottom. The exposure was for 6 hours on slow X-ray film (section 1) and for 24 hours (section 2) and 1 week (section 3) on fast film.

The autoradiogram, which therefore consists of sections with different exposure times, shows that the majority of translational activity migrates at the bottom of the RNA gel (15-20 S). The fact that the translational products of RNA in this size range appear as discrete spots, indicates that these RNAs migrated as discrete bands and that no horizontal spreading of protein occurred during in situ translation and subsequent analysis. In contrast, the products of the larger RNAs appear as horizontal bands. The set of bands starting at a discrete position in the SDS gel, which coincides with the position of 35S RNA in the hybridization profile ( shown at the top), is characteristic of translational products of M-MuLV virion RNA (lane A on the left). Apparently the high molecular weight RNA is predominantly virus specific. As was already evident from the hybridization profile, this RNA appears to be highly degraded. It is believed that the appearance of bands instead of spots in this region of the SDS gel is due not so much to horizontal spreading of protein during or after in situ translation as to translation of RNAs truncated at their 3' ends.

It may therefore be concluded that the two-dimensional blotting procedure according to the invention allows rapid analysis of translational products of a large number of individual RNAs separated on gels.

CLAIMS:

1. A paper for RNA blotting, characterized in that the paper contains ion exchange groups of such a nature and in such amounts per unit of area that RNA can be bound in a reversible fashion by electrostatic interaction.

2. A paper according to claim 1, characterized in that the ion exchange groups are introduced by treating paper or paper starting material with NaOH, triethanolamine and epichlorohydrin.

3. A paper according to claim 2, characterized in that it has been obtained by treating Whatman 1 MM paper or the starting material therefor.

4. A paper according to claim 2 or 3, characterized in that in the treatment of the paper or paper starting material a mixture of triethanolamine and epichlorohydrin is used having a volume ratio triethanolamine/epichlorohydrin of 1 : 2.5 to 1 : 3.5.

5. A paper according to claim 4, characterized in that the volume ratio triethanolamine/epichlorohydrin in the mixture is 1 : 2.8 to 1 : 3.3.

6. A process for making a paper for RNA blotting, characterized in that a paper or paper starting material is provided with ion exchange groups of such a nature and in such amounts per unit of area that RNA can be reversibly bound by electrostatic interaction.

7. A process according to claim 6, characterized in that the ion exchange groups are introduced by treating paper or paper starting material with NaOH, triethanolamine and epichlorohydrin.

8. A process according to claim 7, characterized in that the treatment is carried out at Whatman 1 MM paper or the starting material therefor.

9. A process according to claim 7 or 8, characterized in that in the treatment of the paper or paper starting material a mixture of triethanolamine and epichlorohydrin is used having a volume ratio triethanolamine/epichlorohydrin of 1 : 2.5 to 1 : 3.5.

10. A process according to claim 9, characterized in that the volume ratio triethanolamine/epichlorohydrin in the mixture is 1 : 2.8 to 1 : 3.3.

11. A process according to claim 6, characterized by immersing Whatman 1 MM paper sheets in an aqueous NaOH solution, removing excess NaOH through a yield treatment, then immersing the sheets in a mixture of triethanolamine and epichlorohydrin, in which the volume ratio triethanolamine/epichlorohydrin is 1 : 2.5 to 1 : 3.5, preferably 1 : 2.8 to 1 : 3.3, removing excess mixture through a yield treatment, then immersing the sheets in an aqueous NaOH solution and heating same therein, and subsequently stopping the reaction, washing and finally drying the sheets.

12. A process according to claim 11, characterized in that the aqueous NaOH solution employed is an about 10 M NaOH solution.

13. A process according to claim 11 or 12, characterized in that the first treatment with NaOH is carried out for about 30 min. at about 0°C.

14. A process according to one or more of claims 1-13, characterized in that the second treatment with NaOH is carried out for about 2 hours at about 85-90°C.

15. A process according to one or more of claims 11-14, characterized in that the treatment with a mixture of triethanolamine and epichlorohydrin is carried out for about 15 min. at room temperature.

16. A process according to one or more of claims 11-15, characterized in that the reaction is stopped by placing the sheets in ice-cold 3M KAc-HAc buffer pH 7.4.

17. A process according to one or more of claims 11-16, characterized in that the sheets are washed in few times alternately with 1M HAc and 1M KOH, then with water until neutral and finally with absolute ethanol.

18. A process according to one or more of claims 11-17, characterized in that the sheets are air-dried.

19. A process for blotting RNA, characterized in that the blotting paper employed is a paper according to one or more of claims 1-5 or a paper prepared by using the process according to one or more of claims 6-18.

20. A process according to claim 19, characterized by employing a transfer buffer having a moderate ionic strength and approximately neutral pH.

21. A process according to claim 20, characterized in that the transfer buffer employed is 100 mM KAc-HAc pH 7.4.

22. A process for releasing RNA from paper to which it has been bound by blotting, characterized in that it is released by elution from an RNA blot obtained by using the process according to one or more of claims 19-21.

23. A process according to claim 22, characterized by employing an eluting agent having a high ionic strength and basic pH.

24. A process according to claim 23, characterized in that the eluting agent employed is 30% triethylaminecarbonate pH 9.0.

25. A process for converting messenger RNA into protein by translation, characterized by employing messenger RNA which has been released from a messenger RNA blot by using the process according to one or more of claims 22-24.

26. A process for enzymatically converting RNA into DNA by reversed transcription, characterized by employing RNA which has been released from an RNA blot by using the process according to one or more of claims 22-24.

27. A process for converting messenger RNA into protein by translation, characterized in that the translation is carried out by incubating in a translation mixture a messenger RNA blot obtained by using the process according to one or more of claims 19-21.

28. A process for analyzing a mixture of messenger RNAs by applying electrophoresis, characterized by carrying out an analysis in two dimensions by electrophoresis of the mixture of messenger RNAs in one dimension, blotting of the separated messenger RNAs by using the process according to one or more of claims 19-21, translation of the messenger RNAs in protein by using the process according to claim 27, and electrophoresis of the resulting proteins in the second dimension.

29. A process according to claim 28, characterized by subjecting the mixture of messenger RNAs to agarose gel electrophoresis and the proteins to sodium dodecylsulfate polyacrylamide gel electrophoresis.

30. A process for detecting RNA in blots by hybridization, characterized in that a blot obtained by using the process according to one or more of claims 19-21 is used after the binding of the RNA

to the paper has been strengthened by introducing covalent bonds.

31. A process according to claim 30, characterized by introducing phosphodiester bonds between terminal phosphate groups of the RNA and hydroxyl groups of the paper by means of carbodiimide, after 5' phosphate groups have been formed with P1 nuclease.

FIG.1

FIG.2

FIG.3

0102661

0102661

2/2

FIG.4

RNA GEL ELECTROPHORESIS

ECTEOLA—BLOTTING

CELL—FREE TRANSLATION

SDS—PAGE

gag
Pr 75
gag
Pr65

0102661

# European Patent Office

## EUROPEAN SEARCH REPORT

Application number

EP 83 20 1153

## DOCUMENTS CONSIDERED TO BE RELEVANT

| Category | Citation of document with indication, where appropriate, of relevant passages | Relevant to claim | CLASSIFICATION OF THE APPLICATION (Int. Cl. ³) |
|---|---|---|---|
| X | JOURNAL OF THE AMERICAN CHEMICAL SOCIETY, vol. 78, February 20, 1956, (US) E.A. PETERSON et al.: "Chromatography of proteins. I.Cellulose Ion-exchange adsorbents", pages 751-755 * the whole article * | 1-10 | C 12 N 15/00 C 12 Q 1/68 G 01 N 31/22 |
| | --- | | |
| X | CHEMICAL ABSTRACTS, vol. 66, no. 2, January 9, 1967, page 370, abstract no. 3289z, COLUMBUS, Ohio (US) & Kogyo Kagaku Zasshi 69, 1410-13(1966) M. YOSHIAKI et al.: "Preparation of anion-exchange fabrics based on vinylon fabrics" * the whole abstract * | 1,2 | |
| | --- | | TECHNICAL FIELDS SEARCHED (Int. Cl. ³) |
| A | CHEMICAL ABSTRACTS, vol. 75, no. 4, July 26, 1971, page 128, abstracts no. 22893u, COLUMBUS, Ohio (US) & JP - A - 70 31 690 (SHOWA KOBUNSHI CO., LTD.) (October 14, 1970) * the whole abstract * | | G 01 N C 12 Q D 21 H |
| | ---       -/- | | |

The present search report has been drawn up for all claims

| Place of search | Date of completion of the search | Examiner |
|---|---|---|
| THE HAGUE | 15-12-1983 | OSBORNE H.H. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03.82

| | DOCUMENTS CONSIDERED TO BE RELEVANT | | Page 2 |
|---|---|---|---|
| Category | Citation of document with indication, where appropriate, of relevant passages | Relevant to claim | CLASSIFICATION OF THE APPLICATION (Int. Cl. ³) |
| A | BIOCHEMISTRY, vol. 7, no. 8, August 1968 P.T. GILHAM: "The synthesis of celluloses containing covalently bound nucleotides, polynucleotides, and nucleic acids", pages 2809-2813 * the whole article * | 30,31 | |
| | --- | | |
| A | PROC. NATL. ACAD. SCI. USA, vol. 74, no. 12, December 1977 J.C. ALWINE et al.: "Method for detection of specific RNAs in agarose gels by transfer to diazobenzyloxymethyl-paper and hybridization with DNA probes", pages 5350-5354 * the whole article * | 25-29 | |
| | --- | | TECHNICAL FIELDS SEARCHED (Int. Cl. ³) |
| A | JOURNAL OF VIROLOGY, vol. 41, no. 3, March 1982 B. ADKINS et al.: "Expression of the PRC II avian sarcoma virus genome", pages 767-780 * the whole article * | 25-29 | |
| | ----- | | |

The present search report has been drawn up for all claims

| Place of search THE HAGUE | Date of completion of the search 15-12-1983 | Examiner OSBORNE H.H. |
|---|---|---|

| CATEGORY OF CITED DOCUMENTS | |
|---|---|
| X : particularly relevant if taken alone<br>Y : particularly relevant if combined with another document of the same category<br>A : technological background<br>O : non-written disclosure<br>P : intermediate document | T : theory or principle underlying the invention<br>E : earlier patent document, but published on, or after the filing date<br>D : document cited in the application<br>L : document cited for other reasons<br>& : member of the same patent family, corresponding document |

EPO Form 1503 03 82